# EUROPEAN PATENT SPECIFICATION

(11) **EP 3 206 615 B1**
(45) Date of publication and mention of the grant of the patent: **10.04.2019**
(21) Application number: 15787701.0
(22) Date of filing: 16.10.2015
(51) Int. Cl.: A61B 18/18

(54) **ORGAN RESECTION TOOL**
ORGANRESEKTIONSWERKZEUG
OUTIL DE RÉSECTION D'ORGANE

(30) Priority: 17.10.2014 JP 2014213250
(43) Date of publication of application: 23.08.2017
(73) Proprietor: Neturen Co., Ltd., Tokyo 141-8639 (JP)
(72) Inventor: TANI, Tohru, Otsu-shi Shiga 520-2192 (JP); AKABORI, Hiroya, Otsu-shi Shiga 520-2192 (JP); NAKA, Shigeyuki, Otsu-shi Shiga 520-2192 (JP); YONEDA, Keiichi, Tokyo 141-8639 (JP); FUJIWARA, Fumiaki, Tokyo 141-8639 (JP); MURATA, Yoshiyuki, Tokyo 141-8639 (JP)
(74) Representative: Zimmermann & Partner Patentanwälte mbB
(86) International application number: PCT/JP2015/005242
(87) International publication number: WO 2016/059807

(56) References cited:
- WO-A1-2013/172361
- WO-A1-2013/172361
- WO-A1-2015/072529

## Description

### Technical Field

The present invention relates to an organ resection tool having a brush structure and having a microwave irradiation function.

### Background

Parenchymal organs (such as brains, livers, spleens, kidneys, and pancreases) have a large number of small blood vessels, resulting in a structure that bleeds wherever they are cut. In particular, examples of soft parenchymal organs having a considerable number of capillary blood vessels include livers, pancreases, spleens, and kidneys. Parenchymal organs are harder than soybean curd but do not have strong tissues like hollow organs. Therefore, by applying a vibrator onto a parenchymal organ, the parenchymal organ is sonicated so that cell tissues thereof are dissociated, whereby the parenchymal organ can be resected.

When thinly removing the organ tissues in such organ resection, ultrasonic coagulation cutters and radio-frequency coagulation cutters are primarily being used. However, when removing a portion of an organ at a depth of 1 cm or more, due to thick blood vessels, ultrasonic coagulation cutters and radio-frequency coagulation cutters cannot be used. Thus, when removing a portion of an organ at a deep location, ultrasonic aspirators (such as CUSAs) using radio waves or ultrasonic vibration (cavitation) are being used. However, even with such tools, small bleedings cannot be sufficiently suppressed. Further, while liver cancer is particularly likely to occur in cirrhotic livers, resection performance of ultrasonic aspirators is insufficient for hardened tissues of the cirrhotic livers. Accordingly, in actuality, surgical tools cannot sufficiently resect cirrhotic livers that are most likely to bleed and are found in many cases.

Further, commercially available medical instruments are configured to inject water after disrupting organ tissues with vibrators and to wash out the disrupted organ tissues by aspiration. This operation is repeated to resect a portion of a parenchymal organ with fine blood vessels. The vibrator includes an ultrasonic vibrator configured to generate ultrasonic waves and a transmission member configured to transmit the ultrasonic waves generated by the ultrasonic vibrator to a target (see, e.g., JP2011-206094A).

WO2013/172361A1 discloses an organ resection tool having a brush structure configured to irradiate microwaves and/or a brush structure configured to receive microwaves. In this organ resection tool, the microwaves are applied and received among a plurality of brush elements. That is, the brush elements are limited to materials capable of microwave irradiation.

JP2001-61847A discloses an organ tissue treatment apparatus having an ultrasonic vibrator configured to generate ultrasonic vibration, a probe configured to transmit the ultrasonic vibration from the ultrasonic vibrator to a distal end of the probe, a medium injection means for injecting an ultrasonic-vibration transmission medium between body tissues and the distal end of the probe to transmit the ultrasonic vibration, and a body-tissue removing means for transmitting the ultrasonic vibration from the probe to body tissues through the ultrasonic-vibration transmission medium and removing the body tissues by the ultrasonic vibration transmitted through the ultrasonic-vibration transmission medium. However, it does not disclose or suggest a brush structure of an organ resection tool according to the present invention.

JPS62-001605U discloses a microwave scalpel having a high-frequency coaxial cable to be connected to a microwave generating device at a rear end thereof, needle electrodes connected to a center conductor and an outer conductor of the coaxial cable respectively at a distal end of the coaxial cable, and a rigid blade-shaped dielectric surrounding the needle electrodes such that distal ends of the electrodes are exposed. However, it does not disclose or suggest a brush structure of an organ resection tool according to the present invention.

JP2010-527704A discloses an apparatus for attachment and reinforcement of tissues. The apparatus has an energy applicator positioned adjacent to a first tissue contacting surface and a biopolymer applicator disposed at a second tissue contact surface. The energy applicator is configured to apply an amount of energy to generate heat within a target tissue so as to evaporate intracellular and extracellular water from the target tissue to dry the tissue, and denature at least one of collagen and elastin within the target tissue to attach portions of the target tissue together. The biopolymer applicator is configured for housing a biopolymer material at a location adjacent to the target tissue to receive the heat generated by the energy applied to the target tissue so as to allow the biopolymer material to change phase from a solid state to a molten state, and to allow the biopolymer to fill the dried tissue so as to reinforce the portions of the target tissue attached to one another and provide a hermetic seal once the biopolymer cools and returns to the solid state. However, it does not disclose or suggest a brush structure of an organ resection tool according to the present invention.

JP2008-05515 1A discloses a surgical instrument having a hand piece equipped with a treatment section arranged at the tip side thereof to treat body tissues and including a probe to be supplied with a high frequency current according to a directive and an ultrasonic transducer rigidly secured to the probe and adapted to ultrasonically oscillate the probe; a high frequency drive circuit which supplies a high-frequency current to the probe; and an ultrasonic transducer drive circuit which drives the ultrasonic transducer. The ultrasonic transducer drive circuit controls the amplitude of ultrasonic oscillations of the treatment section according to the magnitude of the impedance value detected by the high frequency drive circuit during high-frequency output. This instrument is provided to prevent body tissues from being scorched by electrodes at a distal end of a scalpel.

### Summary

The related art organ resection tools described above have the following problems.

In a case of performing irradiation and reception of microwaves between brush elements, it is difficult to use materials other than conductors for the brush structures.

An optimal layout of a brush structure for organ resection has not been specified.

To address these problems, according to an aspect of the present invention, an organ resection tool has a brush structure connected directly or indirectly to an insulator and disposed at an optimal position (hereinafter, also referred to as an organ resection tool having a brush structure connected with a nonconductive material).

The related art organ resection tools described above also have the following problems.

During resection or abrading of an organ, adhering of blood or protein to the central portions of the distal ends of brush elements is remarkable.

It is difficult to remove tissues adhered to the brush elements due to heating using microwaves.

An optimal size and features of a brush structure for organ resection have not been specified.

To address these problems, according to another aspect of the present invention, an organ resection tool has a brush structure designed to have a specific arrangement, and/ or a distal end of the brush structure is covered with a film, coated, or diamond-polished (hereinafter, also referred to as an organ resection tool having an optimal brush structure).

The related art organ resection tools described above also have the following problems.

Under a narrow field of view (such as under a view filed of a microscope), since an operation range is restricted, it is necessary to be able to perform resection without moving the tool up and down or left and right.

Since substances adhered to the distal ends of brush elements due to organ resection cause a reduction in coagulation property based on microwave irradiation, it is necessary to remove the adhered substances.

To address these problems, according to another aspect of the present invention, the organ resection tool may be configured such that the distal end of the brush structure is operable to be rotated alternately in left and right directions or in one direction, and/or may additionally have a vibrator and/or a suction device (hereinafter, also referred to as an organ resection tool having a brush structure and an auxiliary device).

According to an aspect of the present invention, an organ resection tool has a first conductor configured to apply microwaves, a second conductor configured to receive the microwaves, an insulator with which at least one of the first conductor and the second conductor is partially or entirely covered, and a brush structure connected directly or indirectly to the insulator or to at least one of the first conductor and the second conductor.

### (Organ Resection Tool with Brush Structure connected to Nonconductive Material)

It becomes possible to intensively irradiate a specific area with microwaves, and thus it is possible to minimize application of microwaves to areas other than resection target tissue. As a result, it is possible to prevent normal tissues from being damaged due to microwave irradiation.

### (Organ Resection Tool with Optimal Brush Structure)

The effect of removing materials having adhered to brush elements is improved, and thus operation time can be shortened. Also, operability is improved due to an improvement in brush element shape, and thus it becomes possible to perform an abrading operation or a resecting operation while maintaining thick blood vessels. Besides, since it is possible to deal with hardness of various organs (in particular, parenchymal organs), it is possible to easily resect organs. Especially, it is possible to disrupt hard tissues such as cirrhotic livers.

### (Organ Resection Tool with Brush Structure and Auxiliary Device)

It is possible to easily remove adhered substances from brush elements by vibrating or rotating the brush elements. Also, under a view field of a microscope, an operator can perform an abrading operation without moving the distal ends of the brush elements up and down or left and right.

Other aspects and advantages of the invention will be apparent from the following description, the drawings and the claims.

### Brief Description of Drawings

Fig. 1 is a cross-sectional view of a distal end portion of an organ resection tool according to an embodiment of the present invention having a brush structure connected to a nonconductive material, the cross-sectional view taken along a microwave irradiation direction.
Fig. 2 is a cross-sectional view of a distal end portion of an organ resection tool according to another embodiment of the present invention having a brush structure connected to a nonconductive material, the cross-sectional view taken along the microwave irradiation direction.
Fig. 3 is a cross-sectional view of a distal end portion of an organ resection tool according to another embodiment of the present invention having a brush structure connected to a nonconductive material, the cross-sectional view taken along the microwave irradiation direction.
Fig. 4 is a cross-sectional view of a distal end portion of an organ resection tool according to another embodiment of the present invention having a brush structure connected to a nonconductive material, the cross-sectional view taken along the microwave irradiation direction.
Fig. 5 is a cross-sectional view of a distal end portion of an organ resection tool according to another embodiment of the present invention having a brush structure connected to a nonconductive material, the cross-sectional view taken along the microwave irradiation direction.
Fig. 6 is a cross-sectional view of a distal end portion of an organ resection tool according to another embodiment of the present invention having a brush structure connected to a nonconductive material, the cross-sectional view taken along the microwave irradiation direction.
Fig. 7 is a cross-sectional view of a distal end portion of an organ resection tool according to another embodiment of the present invention having a brush structure connected to a nonconductive material, the cross-sectional view taken along the microwave irradiation direction.
Fig. 8 is a cross-sectional view of a distal end portion of an organ resection tool according to another embodiment of the present invention having a brush structure connected to a nonconductive material, the cross-sectional view taken along the microwave irradiation direction.
Fig. 9 is a cross-sectional view of a distal end portion of an organ resection tool according to another embodiment of the present invention having a brush structure connected to a nonconductive material, the cross-sectional view taken along the microwave irradiation direction.
Fig. 10 is an external view of an organ resection tool according to another embodiment of the present invention having an optimal brush structure.
Fig. 11 is an external view of an organ resection tool according to another embodiment of the present invention having an optimal brush structure (brush elements omitted).
Fig. 12 is an external view of an organ resection tool according to another embodiment of the present invention having an optimal brush structure.
Fig. 13 is an external view of an organ resection tool according to another embodiment of the present invention having an optimal brush structure.
Fig. 14 is an external view of an organ resection tool having a brush structure and an auxiliary device.
Fig. 15 is a diagram illustrating a cross section of the distal end portion of the organ resection tool of Fig. 6 taken along its longitudinal direction (the microwave irradiation direction).
Fig. 16 is a diagram illustrating a cross section of the distal end portion of the organ resection tool of Fig. 13 taken along its longitudinal direction (the microwave irradiation direction).

### Description of Embodiments

Hereinafter, embodiments of the present invention will be described with reference to the drawings. However, the following embodiments do not limit the scope of the claimed invention. An organ resection tool having a brush structure connected to a nonconductive material, an organ resection tool having an optimal brush structure, and an organ resection tool having a brush structure and an auxiliary device according to one or more embodiments of the present invention has a first conductor (a center conductor or a microwave applying conductor) configured to apply microwaves, a second conductor (an outer conductor or a microwave receiving conductor) configured to receive the microwaves, an insulator with which at least one of the first conductor and the second conductor is partially or entirely covered, and a brush structure connected directly or indirectly to the insulator or to at least one of the first conductor and the second conductor.

In a case in which the brush structure is connected directly or indirectly to at least one of the first conductor and the second conductor, the brush structure has a microwave irradiation probe function.

The center conductor and the outer conductor are configured such that they do not contact each other by providing the insulator or a gap therebetween. Similarly, the microwave applying conductor and the microwave receiving conductor are configured so as not to contact each other.

### (Organ Resection Tool having Brush Structure connected to Nonconductive Material)

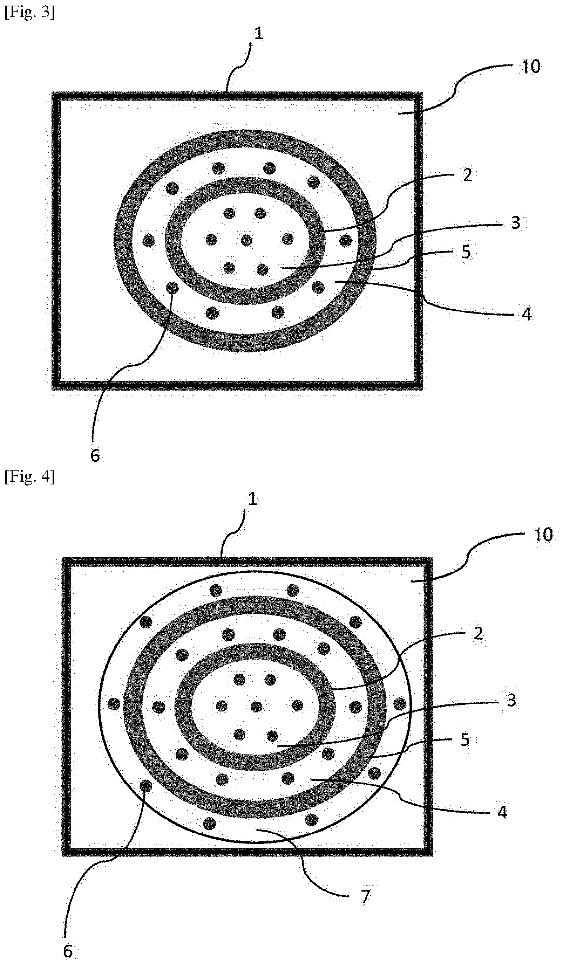

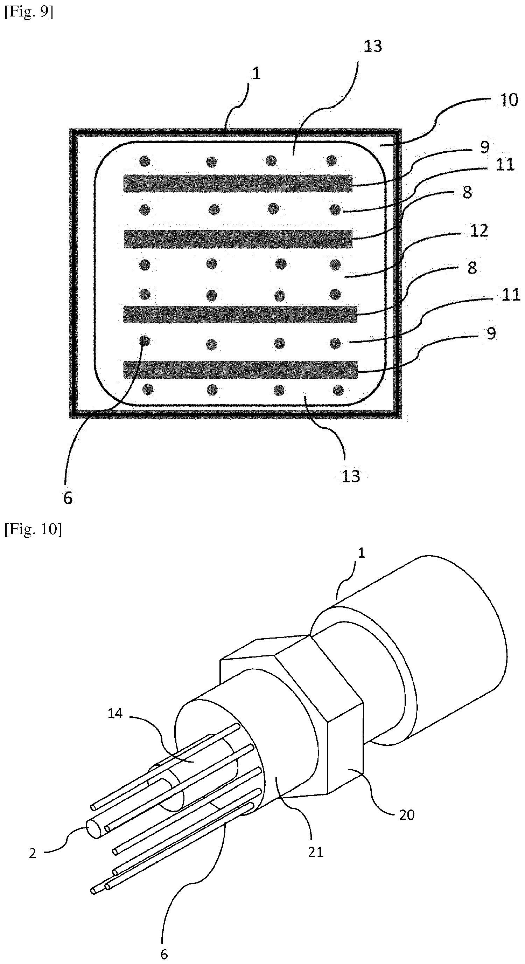

An organ resection tools having a brush structure connected to a nonconductive material according to one or more embodiments of the present invention will be described. The brush structure is connected directly or indirectly to an insulator at the distal end portion of the organ resection tool in a microwave irradiation direction (see, e.g., 3, 4, 7 in Fig.4 and 11, 12, 13 of Fig. 9). The organ resection tool may be configured in the manner described below, so as to intensively irradiate a specific area with microwaves, and to minimize application of the microwaves to areas other than resection target tissue. That is, it is possible to prevent normal tissues from being damaged due to microwave irradiation.

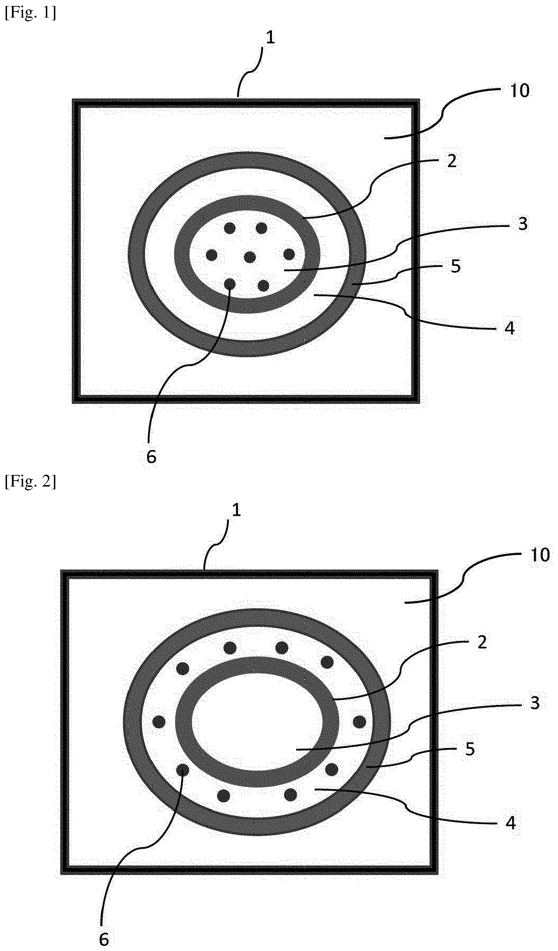

The organ resection tool 1 may be configured such that the first conductor is configured as a center conductor 2 having a hollow cylindrical shape, the insulator has an inner insulator 3 with which an inside of the center conductor 2 is partially or entirely filled and an intermediate insulator 4 with which an outer periphery of the center conductor 2 is partially or entirely covered, the second conductor is configured as an outer conductor 5 with which the intermediate insulator 4 is partially or entirely covered, and the brush structure 6 is connected directly or indirectly to the inner insulator 3 (see Fig. 1).

The organ resection tool 1 may be configured such that the first conductor is configured as a center conductor 2 having a hollow cylindrical shape, the insulator has an inner insulator 3 with which an inside of the center conductor 2 is partially or entirely filled and an intermediate insulator 4 with which an outer periphery of the center conductor 2 is partially or entirely covered, the second conductor is configured as an outer conductor 5 with which the intermediate insulator 4 is partially or entirely covered, and the brush structure 6 is connected directly or indirectly to the intermediate insulator 4 (see Fig. 2).

The organ resection tool 1 may be configured such that the first conductor is configured as a center conductor 2 having a hollow cylindrical shape, the insulator has an inner insulator 3 with which an inside of the center conductor 2 is partially or entirely filled and an intermediate insulator 4 with which an outer periphery of the center conductor 2 is partially or entirely covered, the second conductor is configured as an outer conductor 5 with which the intermediate insulator 4 is partially or entirely covered, and the brush structure 6 is connected directly or indirectly to the inner insulator 3 and to the intermediate insulator 4 (see Fig. 3).

The organ resection tool 1 may be configured such that the first conductor is configured as a center conductor 2 having a hollow cylindrical shape, the insulator has an inner insulator 3 with which an inside of the center conductor 2 is partially or entirely filled and an intermediate insulator 4 with which an outer periphery of the center conductor 2 is partially or entirely covered, the second conductor is configured as an outer conductor 5 with which the intermediate insulator 4 is partially or entirely covered, and the insulator further has an outer insulator 7 with which the outer conductor 5 is partially or entirely covered, and the brush structure 6 is connected directly or indirectly to the inner insulator 3, the intermediate insulator 4, and to the outer insulator 7 (see Fig. 4).

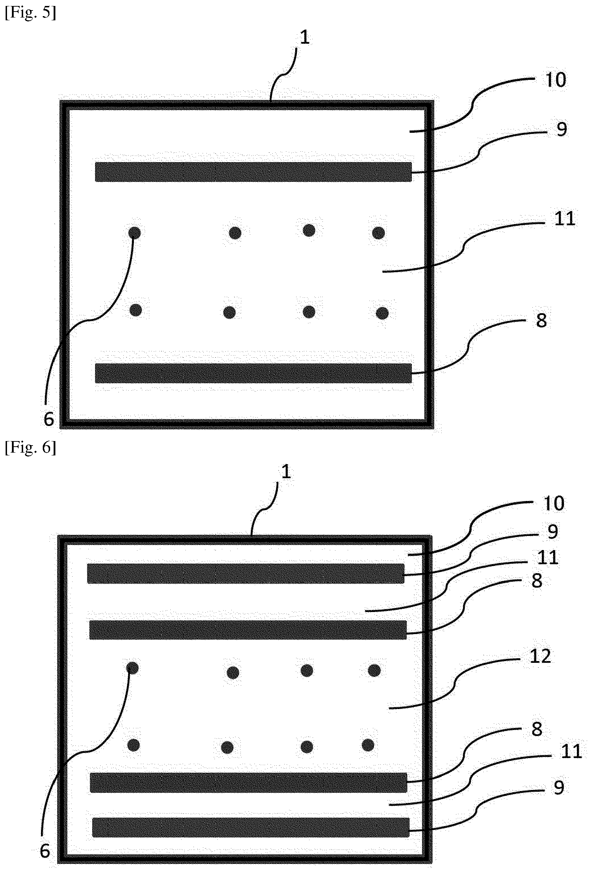

The organ resection tool 1 may be configured such that the first conductor is configured as a microwave applying conductor 8, the second conductor is configured as a microwave receiving conductor 9, the microwave applying conductor 8 and the microwave receiving conductor 9 being arranged parallel to each other, the insulator has a inter-conductor insulator 11 provided between the microwave applying conductor 8 and the microwave receiving conductor 9 in a region defined by two ends of the microwave applying conductor 8 and two ends of the microwave receiving conductor 9, and the brush structure 6 is connected directly or indirectly to the inter-conductor insulator 11 (see Fig. 5).

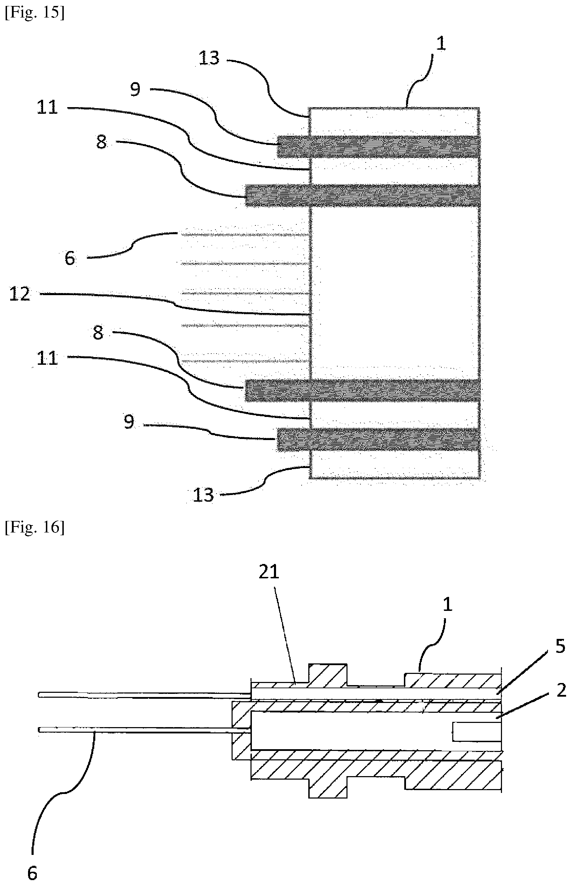

The organ resection tool 1 may be configured such that the first conductor is configured as at least two microwave applying conductors 8, the second conductor is configured as at least two microwave receiving conductors 9, the two microwave receiving conductors 9 being located outward from the two microwave applying conductors 8 in a cross section of the organ resection tool at a distal end of the organ resection tool, the insulator has at least two first inter-conductor insulators 11 provided on outer sides of the two microwave applying conductors 8 in regions between the two microwave applying conductors 8 and the two microwave receiving conductors 9 and a second inter-conductor insulator 12 between the two microwave applying conductors 8 in a region defined by four ends of the two microwave applying conductors 8, and the brush structure 6 is connected directly or indirectly to the second inter-conductor insulator 12 (see Figs. 6 and 15).

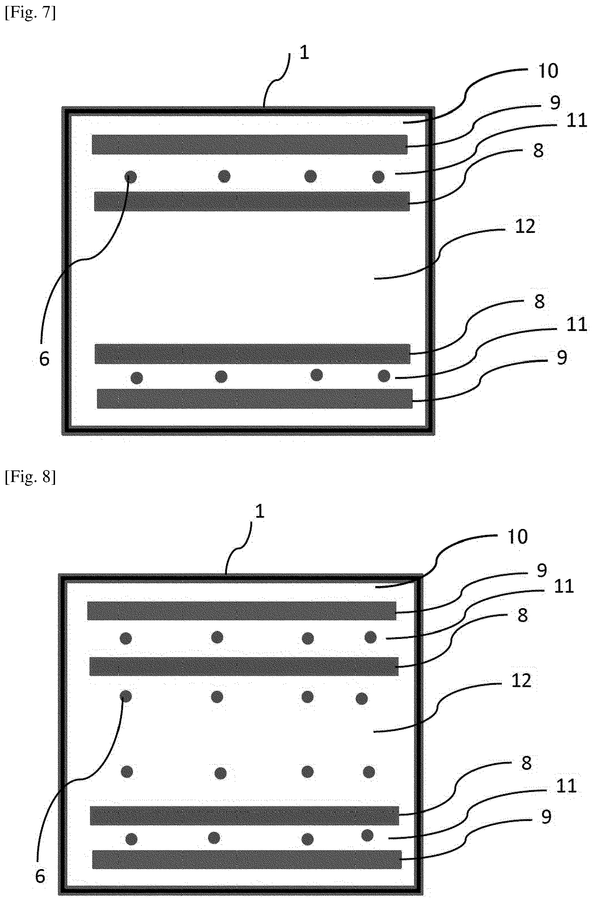

The organ resection tool 1 may be configured such that the first conductor is configured as at least two microwave applying conductors 8, the second conductor is configured as at least two microwave receiving conductors 9, the two microwave receiving conductors 9 being located outward from the two microwave applying conductors 8 in a cross section of the organ resection tool at a distal end of the organ resection tool, the insulator has at least two first inter-conductor insulators 11 provided on outer sides of the two microwave applying conductors 8 in regions between the two microwave applying conductors 8 and the two microwave receiving conductors 9 and a second inter-conductor insulator 12 between the two microwave applying conductors 8 in a region defined by four ends of the two microwave applying conductors 8, and the brush structure 6 is connected directly or indirectly to the two first inter-conductor insulators 11 (see Fig. 7).

The organ resection tool 1 may be configured such that the first conductor is configured as at least two microwave applying conductors 8, the second conductor is configured as at least two microwave receiving conductors 9, the two microwave receiving conductors 9 being located outward from the two microwave applying conductors 8 in a cross section of the organ resection tool at a distal end of the organ resection tool, the insulator has at least two first inter-conductor insulators 11 provided on outer sides of the two microwave applying conductors 8 in regions between the two microwave applying conductors 8 and the two microwave receiving conductors 9 and a second inter-conductor insulator 12 between the two microwave applying conductors 8 in a region defined by four ends of the two microwave applying conductors 8, and the brush structure 6 is connected directly or indirectly to the two first inter-conductor insulators 11 and to the second inter-conductor insulator 12 (see Fig. 8).

The organ resection tool 1 may be configured such that the first conductor is configured as at least two microwave applying conductors 8, the second conductor is configured as at least two microwave receiving conductors 9, the two microwave receiving conductors 9 being located outward from the two microwave applying conductors 8 in a cross section of the organ resection tool at a distal end of the organ resection tool, the insulator has at least two first inter-conductor insulators 11 provided on outer sides of the two microwave applying conductors 8 in regions between the two microwave applying conductors 8 and the two microwave receiving conductors 9, a second inter-conductor insulator 12 between the two microwave applying conductors 8 in a region defined by four ends of the two microwave applying conductors 8, and outer insulators 13 provided in regions on outer sides of the two microwave receiving conductors 9 and the brush structure 6 is connected directly or indirectly to the two first inter-conductor insulators 11, the second inter-conductor insulator 12, and to the outer insulators 13 (see Fig. 9).

### (Organ Resection Tool with Optimal Brush Structure)

An organ resection tool having an optimal brush structure according to one or more embodiments of the present invention is configured such that a brush structure is designed to have a specific arrangement. In addition or alternatively, the distal end of the brush structure is covered with a film, or is subjected to a coating process or a diamond polishing. Specific examples of the organ resection tool with an optimal brush structure will be described below.

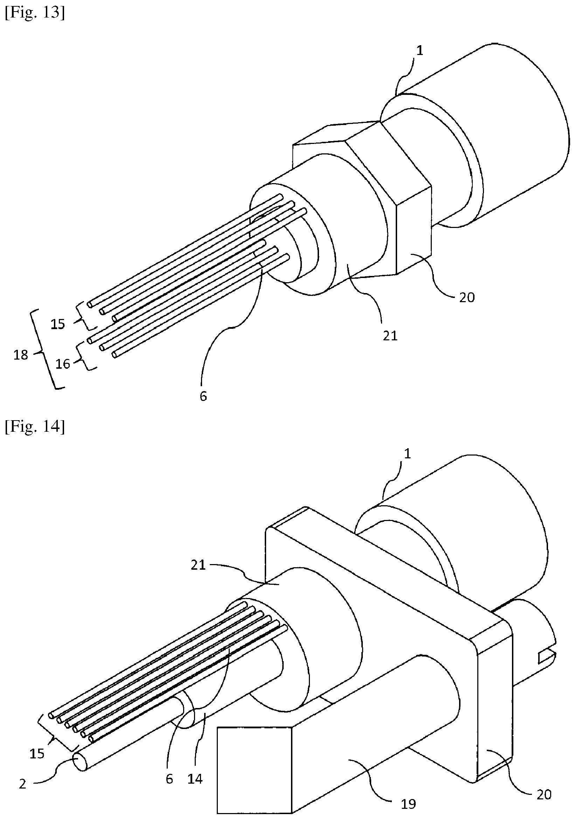

The brush structure 6 of the organ resection tool 1 may have a plurality of brush elements arranged in a straight row 15 and connected directly or indirectly to an outer conductor 5 or to a microwave receiving conductor 9 and/or a plurality of brush elements arranged in a straight row 16 and connected directly or indirectly to a center conductor 2 or to a microwave applying conductor 8 (see Fig. 13). The number of brush elements arranged in a straight row is not particularly limited, and may be 2 to 15, preferably 3 to 13, and more preferably 4 to 10.

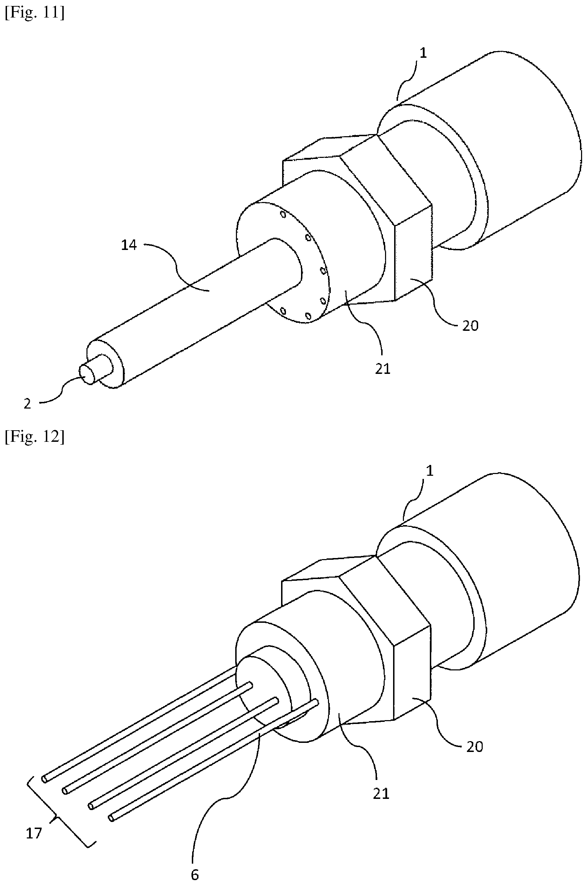

Alternatively, the brush structure 6 may have a plurality of first brush elements connected directly or indirectly to a center conductor 2 or to a microwave applying conductor 8 and a plurality of second brush elements connected directly or indirectly to an outer conductor 5 or to a microwave receiving conductor 9, and the first brush elements and the second brush elements may be arranged in a straight row 17 (see Fig. 12).

The brush structure 6 of the organ resection tool 1 may also be configured such that a plurality of first brush elements connected directly or indirectly to a center conductor 2 or to a microwave applying conductor 8 are arranged in a first straight row 16 and such that a plurality of second brush elements connected directly or indirectly to an outer conductor 5 or to a microwave receiving conductor 9 are arranged in a second straight row 15. The first and second straight rows 18 are preferably substantially parallel to each other (see Figs. 13 and 16).

The first brush elements of the brush structure 6 are connected to the center conductor 2 or to the microwave applying conductor 8 preferably via a brush base 21. Similarly, the second brush elements of the brush structure 6 are connected to the outer conductor 5 or to the microwave receiving conductor 9 preferably via the brush base 21.

The brush structure 6 of the organ resection tool 1 described may be covered with an insulating material 14 (see Figs. 10 and 11).

The distal end of the brush structure 6 of the organ resection tool 1 may be fluorine-coated, silicon-coated, or diamond-polished.

### (Organ Resection Tool with Brush Structure and Auxiliary Device)

An organ resection tool having a brush structure and an auxiliary device according to one or more embodiments of the present invention may be configured such that a distal end of the brush structure is operable to be rotated alternately in left and right directions, or in one direction. The organ resection tool may include a vibrator and/or a suction device. Specific examples of the organ resection tool having such an auxiliary device will be described below.

The organ resection tool 1 may be configured such that the distal end of the brush structure 6 of the organ resection tool 1 is operable to be rotated alternately in left and right directions, or in one direction. The power (or driving mechanism) for the rotation may be a motor or the like, and may be installed inside the organ resection tool 1. By allowing the distal end of the brush structure 6 to rotate alternately in left and right directions or in one direction, it is possible to easily apply microwaves to tissues and to remove (scrape off) the tissues even under a narrow field of view, and thus it is possible to easily remove adhered substances (tissue fragments).

The organ resection tool 1 may have a vibrator to vibrate the distal end of the brush structure 6.

More specifically, the brush structure 6 may be configured to be operable in association with the vibrator. For example, a handle portion connected to the brush structure may be connected to a vibrator, which may be portable or stationary vibrator. The vibrator may be a high-frequency vibrator or an ultrasonic vibrator. Preferably, the vibrator is configured to provide vibrations with a frequency that causes cavitation.

The vibrator may be an ultrasonic vibrator that generates ultrasonic waves, and the ultrasonic waves generated by the ultrasonic vibrator may be transmitted to a target through the brush structure 6. With the vibrator, the organ resection tool 1 can easily apply microwaves to tissues and to remove (scrape off) the tissues even under a narrow field of view, and thus it is possible to easily remove adhered substances (tissue fragments).

The organ resection tool 1 may have a suction device 19 (see Fig. 14). The suction device 19 may include a pipe having a suction port capable of suctioning fragments of cells, and is connected to a suction pump for the suctioning. Preferably, the suction device 19 is configured to inject water. With the suction device 19, the organ resection tool 1 can automatically remove adhered substances (tissue fragments) by suctioning operation. Accordingly, an operator does not need to stop microwave application or tissue removing work in order to remove adhered substances. Further, the suctioning provides effect (cooling effect) of suppressing the temperature rise at a portion of the brush structure functioning as a scalpel. In a case in which the organ resection tool is configured to inject water injection, due to water cooling effect, it is possible to further suppress the temperature rise at the portion of the brush structure functioning as the scalpel.

### (Microwave Irradiation)

A microwave irradiation function (means) is provided preferably by including a microwave transmission unit and a probe. The microwave transmission unit may be provided in a form of a coaxial cable having a center conductor, an insulator, and an outer conductor that are coaxially arranged. Alternatively, the microwave transmission unit may be provided in another form of a microwave applying-receiving structure including a microwave applying conductor having a shape of a rectangular bar, a microwave receiving conductor having a shape of a rectangular bar, and an insulator provided between the microwave applying conductor and the microwave receiving conductor such that the microwave applying conductor and the microwave receiving conductor do not with each other. Frequency of the microwaves is in a range of 900 MHz to 6000 MHz, preferably 2400 MHz to 2500 MHz.

The coaxial cable or other microwave applying-receiving structures are connected to a microwave irradiation device directly or indirectly (through another coaxial cable). The microwave irradiation device is configured to enable a treatment with a small amount of electric power, and is excellent in safety. The electric power is in a range of 1 W to 100 W, preferably 5 W to 60 W, and more preferably 10 W to 40 W. The level of electric power may be adjusted depending on the length of an exposed portion.

### (Coaxial Cable / Microwave Applying-Receiving Structure)

By providing a flexible coaxial cable or microwave applying-receiving structure, the organ resection tool 1 can be inserted into an endoscope and/or into a catheter. The organ resection tool preferably includes a insulative gripping portion to be gripped by an operator during a surgical operation under direct vision such as a laparotomy.

The coaxial cable or the microwave applying-receiving structure includes a center conductor (a microwave applying conductor) made of, for example, phosphor bronze, an insulator in a form of, for example, a shield tube made of Teflon (a trademark) and covering the center conductor, and an outer conductor (a microwave receiving conductor) in a form of, for example, an earth pipe (or grounding pipe) made of brass. The outer side of the coaxial cable may be covered with a shield holder (also referred to as a "guide tube"). The shield holder is preferably made of a non-conductive material (e.g., Teflon (a trademark), a fluorine resin, or a non-magnetic coil such as ceramic).

The preferred diameter of the coaxial body is in a range from 0.3 mm to 5.0 mm.

Examples of the material for the center conductor (the microwave applying conductor) of the coaxial cable (the microwave applying-receiving structure) include copper, bronze, aluminum, and the like. Examples of the material for the insulator include Teflon (trademark), ceramics, and the like. The outer conductor (the microwave receiving conductor) is not particularly limited as long as it is a conductor.

### (Brush Structure)

The brush structures 6 of the organ resection tools 1 are roughly categorized into two types. In a case where a brush structure 6 is connected directly or indirectly to a center conductor, a microwave applying conductor, an outer conductor, or a microwave receiving conductor, the corresponding brush structure is limited to materials capable of microwave irradiation. However, in a case where a brush structure 6 is connected directly or indirectly to an insulator, the corresponding brush structure is not limited to materials capable of microwave irradiation.

In either type, the brush structure 6 has a structure like a brush to contact organs and to scrape off the organs (applying pressure to body tissues).

### (Brush Structure Not Limited to Microwave Irradiating Material)

A brush structure which is not limited to materials capable of microwave irradiation may be a material capable of microwave irradiation. Preferably, the material allows a distal end of the brush structure to be covered with a film, coated, or diamond-polishing.

The diameter ϕ of each brush element of the brush structure 6 is preferably in a range from 0.1 mm to 1.0 mm. The length of each brush element of the brush structure 6 is preferably in a range from 2.0 mm to 30.0 mm. The total number of brush elements of the brush structure 6 is preferably in a range from 1 to 30. The range in which the brush elements of the brush structure 6 are arranged (density) is preferably in a range having a radius of 10.0 mm or less. The Young's modulus of the material of each brush element of the brush structure 6 is preferably in a range from 100 GPa to 220 GPa (e.g., the Young's modulus of brass may be 100 GPa and the Young's modulus of steel may be in a range from 200 GPa to 215 GPa).

By setting two or more, preferably, three or more of the diameter, the length, the density, the total number of brush elements and the Young's modulus of the brush elements in the ranges described above, it is possible to deal with hardness of various organs (in particular, parenchymal organs), and thus it is possible to easily resect the organs.

The portion of the brush structure 6 may be covered, from its connection portion with the insulator 3, 4, 7 or 13 to its distal end portion, by an insulating material (a fluorine resin or a silicon resin) with a film thickness of 1 mm to 5 mm. This coated film makes it easy to remove adhered substances (organ fragments).

The entire brush structure 6 may be fluorine-coated or silicon-coated with a coating thickness of 1 µm to 100 µm, or may be diamond-polished. This makes it possible to prevent adhering of organ fragments (tissues).

The brush structure 6 is configured so as not to damage organs. The brush structure 6 may be configured to be flexible and elastic. The distal end of the brush structure 6 may be configured to be thin (tapered) and be flexibly bendable. Such configurations can cause capillarity. With respect to such configurations, see WO2010/143384A1.

### (Brush Structure Made of Microwave Irradiating Material)

A brush structure 6 made of microwave irradiating material is not particularly limited as long as the material is capable of microwave irradiation and/or capable of receiving microwaves.

For example, various conductive materials made of iron, copper, titanium, stainless steel, phosphor bronze, or brass can be used. Preferably, phosphor bronze, stainless steel, brass, and the like are used.

Hereinafter, organ resection tools according to embodiments of the present invention will be described in detail with reference to specific examples. However, the following examples do not limit scope of the present invention.

### Example 1

### (Organ Resection Tool with Brush Structure connected to Nonconductive Material)

The features of an organ resection tool having a brush structure connected to a nonconductive material were confirmed. Specifically, resection experiments on livers of animals (dogs and pigs) were performed.

The organ resection tool 1 of the embodiment of Fig. 1 was used.

The brush structure 6 of the organ resection tool 1 of Fig. 1 is connected to the non-conductive material. With this brush structure 6, it was possible to intensively irradiate a specific area with microwaves, and thus it was possible to minimize application of microwaves to areas other than resection target tissue. As a result, it was possible to prevent normal tissues from being damaged due to microwave irradiation. Further, due to the features of the brush structure 6, it was possible to deal with hardness of various organs (especially, parenchymal organs).

### Example 2

### (Organ Resection Tool with Optimal Brush Structure)

The features of an organ resection tool having an optimal brush structure were confirmed. Specifically, resection experiments on livers of animals (dogs and pigs) were performed.

The organ resection tool 1 of the embodiment of Fig. 12 and the organ resection tool 1 of the embodiment of Fig. 13 were used.

Each brush structure 6 was covered, from its connection portion with the insulator to its distal end portion, by an insulating material (a silicon resin) with a film thickness of 1 mm to 5 mm. Other features of each brush structure 6 are as follows.
Diameter φ of brush element: 0.1 mm to 1.0 mm
Length of brush element: 2.0 mm to 30.0 mm
Density of brush elements: range having radius of 10.0 mm or less
Total number of brush element: 1 to 30
Young's modulus: 100 GPa to 220 GPa

The brush structure 6 of the organ resection tool 1 of the embodiment of Fig. 12 has the brush elements arranged in a line. The distal ends of the brush elements arranged in a line evenly contacted an operation site, and were able to perform abrasion while performing hemostasis by microwaves.

The brush structure 6 of the organ resection tool 1 of the embodiment of Fig. 13 has the brush elements arranged in two lines. The distal ends of the brush elements arranged in two lines evenly contacted an operation site, and were able to perform abrasion while performing hemostasis by microwaves.

Also, the portion of the brush structure 6 of each organ resection tool 1 was covered, from its connection portion with the insulator to its distal end portion, with a silicon resin, whereby it was easy to remove adhered substances (organ fragments).

Further, the following points were confirmed.

It was possible to easily perform a hemostasis and abrasion operations as compared to conventional organ resection tools.

It was possible to perform a fine operation while maintaining thick blood vessels.

It was possible to easily remove tissues scraped off the operation site and adhered to the organ resection tool, with gauze.

It was possible to easily perform a resection operation as compared to conventional organ resection tools.

### Example 3

### (Organ Resection Tool with Brush Structure and Auxiliary Device)

The features of an organ resection tool having a brush structure and an auxiliary device were confirmed. Specifically, resection experiments on livers of animals (dogs and pigs) were performed.

The organ resection tool 1 of the embodiment of Fig. 14 was used. This organ resection tool 1 has the suction device 19. Other features of the brush structure 6 are as follows.
Diameter ϕ of brush element: 0.1 mm to 1.0 mm
Length of brush element: 2.0 mm to 30.0 mm
Density of brush elements: range having radius of 10.0 mm or less
Total number of brush elements: 1 to 30
Young's modulus: 100 GPa to 220 GPa

The suction device 19 has a pipe that extends through a support base 20, and the pipe has a suction port having a diameter capable of sucking fragments of cells. The pipe is connected to a suction pump.

In the organ resection tool 1 of the embodiment of Fig. 14, organ fragments that adhered to the brush structure 6 were taken into the suction pump of the suction device 19 (the organ fragments were automatically removed by suctioning). As a result, an operator (experimenter) did not need to stop microwave application or tissue removing work for the purpose of removing adhered substances.

### (Conclusion)

As described above, organ resection tools 1 according to one or more embodiments of the present invention (an organ resection tool having a brush structure connected to a nonconductive material, an organ resection tool having an optimal brush structure, an organ resection tool having a brush structure and an auxiliary device) have the following advantages.

### (Organ Resection Tool with Brush Structure connected to Nonconductive Material and/or Optimal Brush Structure)

The effect of removing materials having adhered to the brush elements is improved, and thus operation time decreases.

Operability is improved due to an improvement in brush element shape, and thus it becomes possible to perform an abrading operation or a resecting operation while maintaining thick blood vessels.

Since it is possible to deal with hardness of various organs (in particular, parenchymal organs), it is possible to easily resect organs.

It becomes possible to disrupt hard tissues such as cirrhotic livers.

It becomes possible to intensively irradiate a specific area with a microwave, and thus it is possible to minimize application of a microwave to areas other than resection target tissue. As a result, it is possible to prevent normal tissues from being damaged due to microwave irradiation.

### (Organ Resection Tool having Brush Structure and Auxiliary Device)

It becomes easy to operate a portion including nerves and multiple organs and having been difficult to be abraded or resected.

It is possible to easily remove adhered substances from brush elements by vibrating or rotating the brush elements.

Under a view field of a microscope, an operator can perform an abrading operation without moving the distal ends of the brush elements up and down or left and right.

## Claims

1. An organ resection tool (1) comprising:
a first conductor (2, 8) configured to apply microwaves;
a second conductor (5, 9) configured to receive the microwaves;
an insulator (3, 4, 7, 11, 12, 13) with which at least one of the first conductor (2, 8) and the second conductor (5, 9) is partially or entirely covered; and
a brush structure (6) connected directly or indirectly to the insulator (3, 4, 7, 11, 12, 13) or to at least one of the first conductor (2, 8) and the second conductor (5, 9),
**characterized in that**
the brush structure (6), including a distal end portion of the brush structure (6), is covered with an insulating material, and/or **in that**
a distal end of the brush structure (6) is fluorine-coated, silicon-coated, or diamond-polished.

2. The organ resection tool (1) according to claim 1, wherein the first conductor (2, 8) is configured as a center conductor (2) having a hollow cylindrical shape,
wherein the insulator (3, 4, 7, 11, 12, 13) comprises an inner insulator (3) with which an inside of the center conductor (2) is partially or entirely filled, and an intermediate insulator (4) with which an outer periphery of the center conductor (2) is partially or entirely covered,
wherein the second conductor (5, 9) is configured as an outer conductor (5) with which the intermediate insulator (4) is partially or entirely covered, and
wherein the brush structure (6) is connected directly or indirectly to the inner insulator (3).

3. The organ resection tool (1) according to claim 1, wherein the first conductor (2, 8) is configured as a center conductor (2) having a hollow cylindrical shape,
wherein the insulator (3, 4, 7, 11, 12, 13) comprises an inner insulator (3) with which an inside of the center conductor (2) is partially or entirely filled, and an intermediate insulator (4) with which an outer periphery of the center conductor (2) is partially or entirely covered,
wherein the second conductor (5, 9) is configured as an outer conductor (5) with which the intermediate insulator (4) is partially or entirely covered, and
wherein the brush structure (6) is connected directly or indirectly to the intermediate insulator (4).

4. The organ resection tool (1) according to claim 1, wherein the first conductor (2, 8) is configured as a center conductor (2) having a hollow cylindrical shape,
wherein the insulator (3, 4, 7, 11, 12, 13) comprises an inner insulator (3) with which an inside of the center conductor (2) is partially or entirely filled, and an intermediate insulator (4) with which an outer periphery of the center conductor (2) is partially or entirely covered,
wherein the second conductor (5, 9) is configured as an outer conductor (5) with which the intermediate insulator (4) is partially or entirely covered, and
wherein the brush structure (6) is connected directly or indirectly to the inner insulator (3) and to the intermediate insulator (4).

5. The organ resection tool (1) according to claim 4, wherein the insulator (3, 4, 7, 11, 12, 13) further comprises an outer insulator (7) with which the outer conductor (5) is partially or entirely covered, and
wherein the brush structure (6) is further connected directly or indirectly to the outer insulator (5).

6. The organ resection tool (1) according to claim 1, wherein the first conductor (2, 8) is configured as a microwave applying conductor (8), and the second conductor (5, 9) is configured as a microwave receiving conductor (9), the microwave applying conductor (8) and the microwave receiving conductor (9) being arranged substantially parallel to each other,
wherein the insulator (3, 4, 7, 11, 12, 13) comprises an inter-conductor insulator (11) provided between the microwave applying conductor (8) and the microwave receiving conductor (9) in a region defined by two ends of the microwave applying conductor (8) and two ends of the microwave receiving conductor (9), and
wherein the brush structure (6) is connected directly or indirectly to the inter-conductor insulator (11).

7. The organ resection tool (1) according to claim 1, wherein the first conductor (2, 8) is configured as at least two microwave applying conductors (8), and the second conductor (5, 9) is configured as at least two microwave receiving conductors (9), the two microwave receiving conductors (9) being located outward from the two microwave applying conductors (8) in a cross section of the organ resection tool (1) at a distal end of the organ resection tool (1),
wherein the insulator (3, 4, 7, 11, 12, 13) comprises at least two first inter-conductor insulators (11) provided on outer sides of the two microwave applying conductors (8) in regions between the two microwave applying conductors (8) and the two microwave receiving conductors (9), and a second inter-conductor (12) insulator provided between the two microwave applying conductors (8) in a region defined by four ends of the two microwave applying conductors (8), and
wherein the brush structure (6) is connected directly or indirectly to the second inter-conductor insulator (12).

8. The organ resection tool (1) according to claim 1, wherein the first conductor (2, 8) is configured as at least two microwave applying conductors (8), and the second conductor (5, 9) is configured as at least two microwave receiving conductors (9), the two microwave receiving conductors (9) being located outward from the two microwave applying conductors (8) in a cross section of the organ resection tool (1) at a distal end of the organ resection tool (1),
wherein the insulator (3, 4, 7, 11, 12, 13) comprises at least two first inter-conductor insulators (11) provided on outer sides of the two microwave applying conductors (8) in regions between the two microwave applying conductors (8) and the two microwave receiving conductors (9), and a second inter-conductor insulator (12) provided between the two microwave applying conductors (8) in a region defined by four ends of the two microwave applying conductors (8), and
wherein the brush structure (6) is connected directly or indirectly to the two first inter-conductor insulators (11).

9. The organ resection tool (1) according to claim 8, wherein the brush structure (6) is further connected directly or indirectly to the second inter-conductor insulator (12).

10. The organ resection tool (1) according to claim 9, wherein the insulator (3, 4, 7, 11, 12, 13) further comprises outer insulators (13) provided in regions on outer sides of the two microwave receiving conductors (9), and
wherein the brush structure (6) is further connected directly or indirectly to the outer insulators (9).

11. The organ resection tool (1) according to claim 1, wherein the brush structure (6) comprises a plurality of brush elements connected directly or indirectly to the first conductor (2, 8), wherein the brush elements are arranged in a straight row (16).

12. The organ resection tool (1) according to claim 1, wherein the brush structure (6) comprises a plurality of brush elements connected directly or indirectly to the second conductor, wherein the brush elements are arranged in a straight row (16).

13. The organ resection tool (1) according to claim 1, wherein the brush structure (6) comprises a plurality of first brush elements connected directly or indirectly to the first conductor (2, 8) and a plurality of second brush elements connected directly or indirectly to the second conductor (5, 9), wherein the first brush elements and the second brush elements are arranged in a straight row (15).

14. The organ resection tool (1) according to claim 1, wherein the brush structure (6) comprises a plurality of first brush elements connected directly or indirectly to the first conductor (2, 8), and a plurality of second brush elements connected directly or indirectly to the second conductor (5, 9), wherein the first brush elements are arranged in a first straight row (16) and the second brush elements are arranged in a second straight row (15), the first and second straight rows (18) being substantially parallel to each other.

15. The organ resection tool (1) according to any one of claims 1 to 14, wherein a distal end of the brush structure (6) is operable to be rotated alternately in two directions or in one direction.

16. The organ resection tool (1) according to any one of claims 1 to 15, further comprising a vibrator to vibrate a distal end of the brush structure.

17. The organ resection tool (1) according to any one of claims 1 to 16, further comprising a suction device (19).

## Patentansprüche

1. Organresektionswerkzeug (1), Folgendes umfassend:
einen ersten Leiter (2, 8), der dazu ausgelegt ist, Mikrowellen anzulegen;
einen zweiten Leiter (5, 9), der dazu ausgelegt ist, die Mikrowellen zu empfangen;
einen Isolator (3, 4, 7, 11, 12, 13), mit dem der erste Leiter (2, 8) und/oder zweite Leiter (5, 9) teilweise oder gänzlich bedeckt ist; und
eine Bürstenstruktur (6), die direkt oder indirekt an den Isolator (3, 4, 7, 11, 12, 13) oder an den ersten Leiter (2, 8) und/oder zweiten Leiter (5, 9) angeschlossen ist,
**dadurch gekennzeichnet, dass**
die Bürstenstruktur (6), einschließlich eines distalen Endabschnitts der Bürstenstruktur (6), mit einem Isoliermaterial bedeckt ist, und/oder, dass
ein distales Ende der Bürstenstruktur (6) fluorbeschichtet, siliciumbeschichtet oder diamantpoliert ist.

2. Organresektionswerkzeug (1) nach Anspruch 1, wobei der erste Leiter (2, 8) als Mittelleiter (2) mit einer hohlzylindrischen Form ausgelegt ist,
wobei der Isolator (3, 4, 7, 11, 12, 13) einen Innenisolator (3), mit dem ein Innenraum des Mittelleiters (2) teilweise oder gänzlich gefüllt ist, und einen Zwischenisolator (4) umfasst, mit dem ein Außenumfang des Mittelleiters (2) teilweise oder gänzlich bedeckt ist,
wobei der zweite Leiter (5, 9) als Außenleiter (5) ausgelegt ist, mit dem der Zwischenisolator (4) teilweise oder gänzlich bedeckt ist, und
wobei die Bürstenstruktur (6) direkt oder indirekt an den Innenisolator (3) angeschlossen ist.

3. Organresektionswerkzeug (1) nach Anspruch 1, wobei der erste Leiter (2, 8) als Mittelleiter (2) mit einer hohlzylindrischen Form ausgelegt ist,
wobei der Isolator (3, 4, 7, 11, 12, 13) einen Innenisolator (3), mit dem ein Innenraum des Mittelleiters (2) teilweise oder gänzlich gefüllt ist, und einen Zwischenisolator (4) umfasst, mit dem ein Außenumfang des Mittelleiters (2) teilweise oder gänzlich bedeckt ist,
wobei der zweite Leiter (5, 9) als Außenleiter (5) ausgelegt ist, mit dem der Zwischenisolator (4) teilweise oder gänzlich bedeckt ist, und
wobei die Bürstenstruktur (6) direkt oder indirekt an den Zwischenisolator (4) angeschlossen ist.

4. Organresektionswerkzeug (1) nach Anspruch 1, wobei der erste Leiter (2, 8) als Mittelleiter (2) mit einer hohlzylindrischen Form ausgelegt ist,
wobei der Isolator (3, 4, 7, 11, 12, 13) einen Innenisolator (3), mit dem ein Innenraum des Mittelleiters (2) teilweise oder gänzlich gefüllt ist, und einen Zwischenisolator (4) umfasst, mit dem ein Außenumfang des Mittelleiters (2) teilweise oder gänzlich bedeckt ist,
wobei der zweite Leiter (5, 9) als Außenleiter (5) ausgelegt ist, mit dem der Zwischenisolator (4) teilweise oder gänzlich bedeckt ist, und
wobei die Bürstenstruktur (6) direkt oder indirekt an den Innenisolator (3) und den Zwischenisolator (4) angeschlossen ist.

5. Organresektionswerkzeug (1) nach Anspruch 4, wobei der Isolator (3, 4, 7, 11, 12, 13) ferner einen Außenisolator (7) umfasst, mit dem der Außenleiter (5) teilweise oder gänzlich bedeckt ist, und wobei
die Bürstenstruktur (6) ferner direkt oder indirekt an den Außenisolator (7) angeschlossen ist.

6. Organresektionswerkzeug (1) nach Anspruch 1, wobei der erste Leiter (2, 8) als Mikrowellen anlegender Leiter (8) ausgelegt ist, und der zweite Leiter (5, 9) als Mikrowellen empfangender Leiter (9) ausgelegt ist, wobei der Mikrowellen anlegende Leiter (8) und der Mikrowellen empfangende Leiter (9) im Wesentlichen parallel zueinander angeordnet sind,
wobei der Isolator (3, 4, 7, 11, 12, 13) einen Zwischenleiterisolator (11) umfasst, der zwischen dem Mikrowellen anlegenden Leiter (8) und dem Mikrowellen empfangenden Leiter (9) in einem Bereich vorgesehen ist, der durch zwei Enden des Mikrowellen anlegenden Leiters (8) und zwei Enden des Mikrowellen empfangenden Leiters (9) festgelegt ist, und
wobei die Bürstenstruktur (6) direkt oder indirekt an den Zwischenleiterisolator (11) angeschlossen ist.

7. Organresektionswerkzeug (1) nach Anspruch 1, wobei der erste Leiter (2, 8) als mindestens zwei Mikrowellen anlegende Leiter (8) ausgelegt ist, und der zweite Leiter (5, 9) als mindestens zwei Mikrowellen empfangende Leiter (9) ausgelegt ist, wobei sich die zwei Mikrowellen empfangenden Leiter (9) in einem Querschnitt des Organresektionswerkzeugs (1) außerhalb der zwei Mikrowellen anlegenden Leiter (8) an einem distalen Ende des Organresektionswerkzeugs (1) befinden,
wobei der Isolator (3, 4, 7, 11, 12, 13) mindestens zwei erste Zwischenleiterisolatoren (11), die an Außenseiten der zwei Mikrowellen anlegenden Leiter (8) in Bereichen zwischen den zwei Mikrowellen anlegenden Leitern (8) und den zwei Mikrowellen empfangenden Leitern (9) vorgesehen sind, und einen zweiten Zwischenleiterisolator (12) umfasst, der zwischen den zwei Mikrowellen anlegenden Leitern (8) in einem Bereich vorgesehen ist, der durch vier Enden der zwei Mikrowellen anlegenden Leiter (8) festgelegt ist, und
wobei die Bürstenstruktur (6) direkt oder indirekt an den zweiten Zwischenleiterisolator (12) angeschlossen ist.

8. Organresektionswerkzeug (1) nach Anspruch 1, wobei der erste Leiter (2, 8) als mindestens zwei Mikrowellen anlegende Leiter (8) ausgelegt ist, und der zweite Leiter (5, 9) als mindestens zwei Mikrowellen empfangende Leiter (9) ausgelegt ist, wobei sich die zwei Mikrowellen empfangenden Leiter (9) in einem Querschnitt des Organresektionswerkzeugs (1) außerhalb der zwei Mikrowellen anlegenden Leiter (8) an einem distalen Ende des Organresektionswerkzeugs (1) befinden,
wobei der Isolator (3, 4, 7, 11, 12, 13) mindestens zwei erste Zwischenleiterisolatoren (11), die an Außenseiten der zwei Mikrowellen anlegenden Leiter (8) in Bereichen zwischen den zwei Mikrowellen anlegenden Leitern (8) und den zwei Mikrowellen empfangenden Leitern (9) vorgesehen sind, und einen zweiten Zwischenleiterisolator (12) umfasst, der zwischen den zwei Mikrowellen anlegenden Leitern (8) in einem Bereich vorgesehen ist, der durch vier Enden der zwei Mikrowellen anlegenden Leiter (8) festgelegt ist, und
wobei die Bürstenstruktur (6) direkt oder indirekt an die zwei ersten Zwischenleiterisolatoren (11) angeschlossen ist.

9. Organresektionswerkzeug (1) nach Anspruch 8, wobei die Bürstenstruktur (6) ferner direkt oder indirekt an den zweiten Zwischenleiterisolator (12) angeschlossen ist.

10. Organresektionswerkzeug (1) nach Anspruch 9, wobei der Isolator (3, 4, 7, 11, 12, 13) ferner Außenisolatoren (13) umfasst, die in Bereichen auf Außenseiten der zwei Mikrowellen empfangenden Leiter (9) vorgesehen sind, und
wobei die Bürstenstruktur (6) ferner direkt oder indirekt an die Außenisolatoren (13) angeschlossen ist.

11. Organresektionswerkzeug (1) nach Anspruch 1, wobei die Bürstenstruktur (6) mehrere Bürstenelemente umfasst, die direkt oder indirekt an den ersten Leiter (2, 8) angeschlossen sind, wobei die Bürstenelemente in einer geraden Reihe (16) angeordnet sind.

12. Organresektionswerkzeug (1) nach Anspruch 1, wobei die Bürstenstruktur (6) mehrere Bürstenelemente umfasst, die direkt oder indirekt an den zweiten Leiter angeschlossen sind, wobei die Bürstenelemente in einer geraden Reihe (16) angeordnet sind.

13. Organresektionswerkzeug (1) nach Anspruch 1, wobei die Bürstenstruktur (6) mehrere erste Bürstenelemente, die direkt oder indirekt an den ersten Leiter (2, 8) angeschlossen sind, und mehrere zweite Bürstenelemente umfasst, die direkt oder indirekt an den zweiten Leiter (5, 9) angeschlossen sind, wobei die ersten Bürstenelemente und die zweiten Bürstenelemente in einer geraden Reihe (15) angeordnet sind.

14. Organresektionswerkzeug (1) nach Anspruch 1, wobei die Bürstenstruktur (6) mehrere erste Bürstenelemente, die direkt oder indirekt an den ersten Leiter (2, 8) angeschlossen sind, und mehrere zweite Bürstenelemente umfasst, die direkt oder indirekt an den zweiten Leiter (5, 9) angeschlossen sind, wobei die ersten Bürstenelemente in einer ersten geraden Reihe (16) angeordnet sind, und die zweiten Bürstenelemente in einer zweiten geraden Reihe (15) angeordnet sind, wobei die erste und zweite Reihe (18) im Wesentlichen parallel zueinander sind.

15. Organresektionswerkzeug (1) nach einem der Ansprüche 1 bis 14, wobei ein distales Ende der Bürstenstruktur (6) bedienbar ist, um abwechselnd in zwei Richtungen oder in eine Richtung gedreht zu werden.

16. Organresektionswerkzeug (1) nach einem der Ansprüche 1 bis 15, ferner einen Schwingungserzeuger umfassend, um ein distales Ende der Bürstenstruktur in Schwingung zu versetzen.

17. Organresektionswerkzeug (1) nach einem der Ansprüche 1 bis 16, ferner eine Absaugvorrichtung (19) umfassend.

## Revendications

1. Outil de résection d'organe (1) comprenant :
un premier conducteur (2, 8) configuré pour appliquer des microondes ;
un deuxième conducteur (5, 9) configuré pour recevoir les microondes ;
un isolant (3, 4, 7, 11, 12, 13) dont au moins l'un du premier conducteur (2, 8) et du deuxième conducteur (5, 9) est partiellement ou entièrement recouvert ; et une structure à balai (6) directement ou indirectement connectée à l'isolant (3, 4, 7, 11, 12, 13) ou à au moins l'un du premier conducteur (2, 8) et du deuxième conducteur (5, 9),
**caractérisé en ce que**
la structure à balai (6), incluant une partie d'extrémité distale de la structure à balai (6), est recouverte d'un matériau isolant, et/ou **en ce que**
une extrémité distale de la structure à balai (6) est revêtue de fluorine, revêtue de silicium, ou polie au diamant.

2. L'outil de résection d'organe (1) selon la revendication 1, sachant que le premier conducteur (2, 8) est configuré comme conducteur central (2) ayant une forme cylindrique creuse,
sachant que l'isolant (3, 4, 7, 11, 12, 13) comprend un isolant intérieur (3) dont un intérieur du conducteur central (2) est partiellement ou entièrement rempli, et un isolant intermédiaire (4) dont une périphérie extérieure du conducteur central (2) est partiellement ou entièrement recouverte,
sachant que le deuxième conducteur (5, 9) est configuré comme conducteur extérieur (5) dont l'isolant intermédiaire (4) est partiellement ou entièrement recouvert, et
sachant que la structure à balai (6) est connectée directement ou indirectement à l'isolant intérieur (3).

3. L'outil de résection d'organe (1) selon la revendication 1, sachant que le premier conducteur (2, 8) est configuré comme conducteur central (2) ayant une forme cylindrique creuse,
sachant que l'isolant (3, 4, 7, 11, 12, 13) comprend un isolant intérieur (3) dont un intérieur du conducteur central (2) est partiellement ou entièrement rempli, et un isolant intermédiaire (4) dont une périphérie extérieure du conducteur central (2) est partiellement ou entièrement recouverte,
sachant que le deuxième conducteur (5, 9) est configuré comme conducteur extérieur (5) dont l'isolant intermédiaire (4) est partiellement ou entièrement recouvert, et
sachant que la structure à balai (6) est directement ou indirectement connectée à l'isolant intermédiaire (4).

4. L'outil de résection d'organe (1) selon la revendication 1, sachant que le premier conducteur (2, 8) est configuré comme conducteur central (2) ayant une forme cylindrique creuse,
sachant que l'isolant (3, 4, 7, 11, 12, 13) comprend un isolant intérieur (3) dont un intérieur du conducteur central (2) est partiellement ou entièrement rempli, et un isolant intermédiaire (4) dont une périphérie extérieure du conducteur central (2) est partiellement ou entièrement recouverte,
sachant que le deuxième conducteur (5, 9) est configuré comme conducteur extérieur (5) dont l'isolant intermédiaire (4) est partiellement ou entièrement recouvert, et
sachant que la structure à balai (6) est directement ou indirectement connectée à l'isolant intérieur (3) et à l'isolant intermédiaire (4).

5. L'outil de résection d'organe (1) selon la revendication 4, sachant que l'isolant (3, 4, 7, 11, 12, 13) comprend en outre un isolant extérieur (7) dont le conducteur extérieur (5) est partiellement ou entièrement recouvert, et
sachant que la structure à balai (6) est en outre directement ou indirectement connectée à l'isolant extérieur (7).

6. L'outil de résection d'organe (1) selon la revendication 1, sachant que le premier conducteur (2, 8) est configuré comme conducteur d'application de microondes (8), et le deuxième conducteur (5, 9) est configuré comme conducteur de réception de microondes (9), le conducteur d'application de microondes (8) et le conducteur de réception de microondes (9) étant agencés de manière sensiblement parallèle l'un à l'autre,
sachant que l'isolant (3, 4, 7, 11, 12, 13) comprend un isolant inter-conducteurs (11) prévu entre le conducteur d'application de microondes (8) et le conducteur de réception de microondes (9) dans une zone définie par deux extrémités du conducteur d'application de microondes (8) et deux extrémités du conducteur de réception de microondes (9), et
sachant que la structure à balai (6) est directement ou indirectement connectée à l'isolant inter-conducteurs (11).

7. L'outil de résection d'organe (1) selon la revendication 1, sachant que le premier conducteur (2, 8) est configuré comme au moins deux conducteurs d'application de microondes (8), et le deuxième conducteur (5, 9) est configuré comme au moins deux conducteurs de réception de microondes (9), les deux conducteurs de réception de microondes (9) étant situés à l'extérieur des deux conducteurs d'application de microondes (8) dans une section transversale de l'outil de résection d'organe (1) à une extrémité distale de l'outil de résection d'organe (1),
sachant que l'isolant (3, 4, 7, 11, 12, 13) comprend au moins deux premiers isolants inter-conducteurs (11) prévus sur des côtés extérieurs des deux conducteurs d'application de microondes (8) dans des zones entre les deux conducteurs d'application de microondes (8) et les deux conducteurs de réception de microondes (9), et un deuxième isolant inter-conducteurs (12) prévu entre les deux conducteurs d'application de microondes (8) dans une zone définie par quatre extrémités des conducteurs d'application de microondes (8), et
sachant que la structure à balai (6) est directement ou indirectement connectée au deuxième isolant inter-conducteurs (12).

8. L'outil de résection d'organe (1) selon la revendication 1, sachant que le premier conducteur (2, 8) est configuré comme au moins deux conducteurs d'application de microondes (8), et le deuxième conducteur (5, 9) est configuré comme au moins deux conducteurs de réception de microondes (9), les deux conducteurs de réception de microondes (9) étant situés à l'extérieur des deux conducteurs d'application de microondes (8) dans une section transversale de l'outil de résection d'organe (1) à une extrémité distale de l'outil de résection d'organe (1),
sachant que l'isolant (3, 4, 7, 11, 12, 13) comprend au moins deux premiers isolants inter-conducteurs (11) prévus sur des côtés extérieurs des deux conducteurs d'application de microondes (8) dans des zones entre les deux conducteurs d'application de microondes (8) et les deux conducteurs de réception de microondes (9), et un deuxième isolant inter-conducteurs (12) prévu entre les deux conducteurs d'application de microondes (8) dans une zone définie par quatre extrémités des conducteurs d'application de microondes (8), et
sachant que la structure à balai (6) est directement ou indirectement connectée aux deux premiers isolants inter-conducteurs (11).

9. L'outil de résection d'organe (1) selon la revendication 8, sachant que la structure à balai (6) est en outre directement ou indirectement connectée au deuxième isolant inter-conducteurs (12).

10. L'outil de résection d'organe (1) selon la revendication 9, sachant que l'isolant (3, 4, 7, 11, 12, 13) comprend en outre des isolants extérieurs (13) prévus dans des zones sur des côtés extérieurs des deux conducteurs de réception de microondes (9), et
sachant que la structure à balai (6) est en outre directement ou indirectement connectée aux isolants extérieurs (13).

11. L'outil de résection d'organe (1) selon la revendication 1, sachant que la structure à balai (6) comprend une pluralité d'éléments de balai directement ou indirectement connectés au premier conducteur (2, 8), sachant que les éléments de balai sont agencés en une rangée (16) droite.

12. L'outil de résection d'organe (1) selon la revendication 1, sachant que la structure à balai (6) comprend une pluralité d'éléments de balai directement ou indirectement connectés au deuxième conducteur, sachant que les éléments de balai sont agencés en une rangée (16) droite.

13. L'outil de résection d'organe (1) selon la revendication 1, sachant que la structure à balai (6) comprend une pluralité de premiers éléments de balai directement ou indirectement connectés au premier conducteur (2, 8) et une pluralité de deuxièmes éléments de balai directement ou indirectement connectés au deuxième conducteur (5, 9), sachant que les premiers éléments de balai et les deuxièmes éléments de balai sont agencés en une rangée (15) droite.

14. L'outil de résection d'organe (1) selon la revendication 1, sachant que la structure à balai (6) comprend une pluralité de premiers éléments de balai directement ou indirectement connectés au premier conducteur (2, 8), et une pluralité de deuxièmes éléments de balai directement ou indirectement connectés au deuxième conducteur (5, 9), sachant que les premiers éléments de balai sont agencés en une première rangée (16) droite et les deuxièmes éléments de balai sont agencés en une deuxième rangée (15) droite, la première et la deuxième rangée (18) droite étant sensiblement parallèles l'une à l'autre.

15. L'outil de résection d'organe (1) selon l'une quelconque des revendications 1 à 14, sachant qu'une extrémité distale de la structure à balai (6) est manoeuvrable pour être tournée en alternance dans deux directions ou dans une direction.

16. L'outil de résection d'organe (1) selon l'une quelconque des revendications 1 à 15, comprenant en outre un vibrateur pour faire vibrer une extrémité distale de la structure à balai.

17. L'outil de résection d'organe (1) selon l'une quelconque des revendications 1 à 16, comprenant en outre un dispositif d'aspiration (19).
